# EUROPEAN PATENT SPECIFICATION

(11) **EP 0 555 605 B1**
(45) Date of publication and mention of the grant of the patent: **04.10.1995**
(21) Application number: 92403441.6
(22) Date of filing: 17.12.1992
(51) Int. Cl.: B01L 3/00, C12Q 1/22, B65D 51/16

(54) **Device for checking the sterility of a fluid**
Vorrichtung zur Kontrolle der Sterilität eines Fluids
Dispositif de vérification de la stérilité d'un fluide

(30) Priority: 13.02.1992 FR 9201605
(43) Date of publication of application: 18.08.1993
(73) Proprietor: MILLIPORE S.A., F-67120 Molsheim (FR)
(72) Inventor: Lemonnier, Jean, F-78110 Le Vesinet (FR)
(74) Representative: Rinuy, Santarelli

(56) References cited:
- EP-A- 0 150 775
- EP-A- 0 403 380
- DE-A- 2 847 929
- FR-A- 2 540 992
- FR-A- 2 545 374
- US-A- 4 215 198
- US-A- 4 392 055

## Description

The present invention concerns a single-use device for checking the sterility of a sample of fluid, in particular fluid containing antibiotics, as described in the French patents 2 290 496 and 2 540 992.

Until now prior art devices used for such checking and described in the above-mentioned patents have all comprised a closed container incorporating at least two filter membranes, a hydrophilic membrane for sampling the fluid to be analysed, preferably under pressure, and a hydrophobic membrane to enable the required gaseous equilibrium between the inside and the outside of the container to be achieved during the operations carried out to check the sterility of the sample.

An object of the present invention is to reduce the constraints of the cited patents by proposing a more economical device incorporating a single filter membrane that is easy to weld.

The device in accordance with the present invention comprises a transparent rigid plastic material container including an upper part, a disk-shape hydrophilic microporous filter membrane and a lower part fastened to a drainage support, the two parts of the container and the microporous filter membrane disposed between the lower edge of the upper part and the upper edge of the lower part of the container being welded together in a fluid-tight manner at their peripheries.

This device is characterised in that the upper part of the container has a frustoconical section whose base delimits the filter surface area of the membrane and whose other end has a smaller diameter, and a funnel-shape part widening from the smaller diameter end of the frustoconical part to an open upper end whose diameter is greater than said smaller diameter.

And said device in accordance with the invention is further characterised in that the open upper end of the container is adapted to be closed by a flexible plastics material lid adapted to be deformed by the pressure inside the container during incubation and provided with at least one vent hole to vent excess pressure without contamination from the outside.

The lid may be fitted to the container by a system of lid fastening and sealing lips in conjunction with a ring provided at the open end of the container enabling air to enter when filtering the fluid sample whose sterility is to be checked and air to exit during incubation of a liquid culture medium in the container.

The present invention will now be described with reference to a preferred embodiment shown in the appended drawings, in which:
- figure 1 is a view in elevation of the device in accordance with the invention partially cut away at the bottom and partially in cross-section at the top;
- figure 2 is a bottom view of the device from figure 1;
- figure 3 is an enlarged cross-section showing the position of the lid on the container during filtering of the sample as shown in figure 1;
- figure 4 is a view in elevation similar to that of figure 1 except that the position of the lid on the container is different, being that for incubating a culture medium;
- figure 5 is a view in cross-section to a larger scale showing the same position of the lid on the container as figure 4;
- figure 6 shows the welding of the microporous filter membrane to the upper part of the container, the membrane being in the form of a disk held by a vacuum against an interior support (23);
- figure 7 shows the welding of the lower part of the container fastened to a drainage support to the upper part; and
- figure 8 shows the combination of the lower and upper parts of the container welded to the microporous filter membrane sandwiched between them.

The sterility checking device shown in the appended figures comprises a container (1) and a lid (2).

The container 1 comprises an upper part (3), a microporous filter membrane (4) and a lower part (5).

The upper part (3) has a frustoconical section (6) which has at the lower end a circular ring (13) to which the microporous filter membrane (4) is welded and a smaller diameter upper end (7) which forms the part of the container with the smallest diameter.

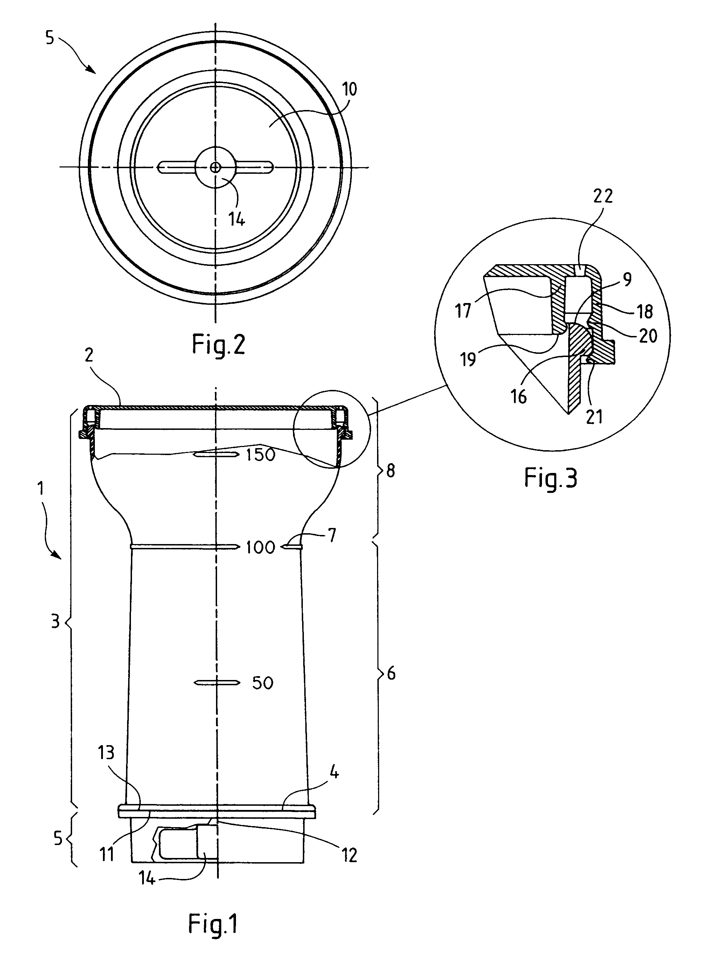

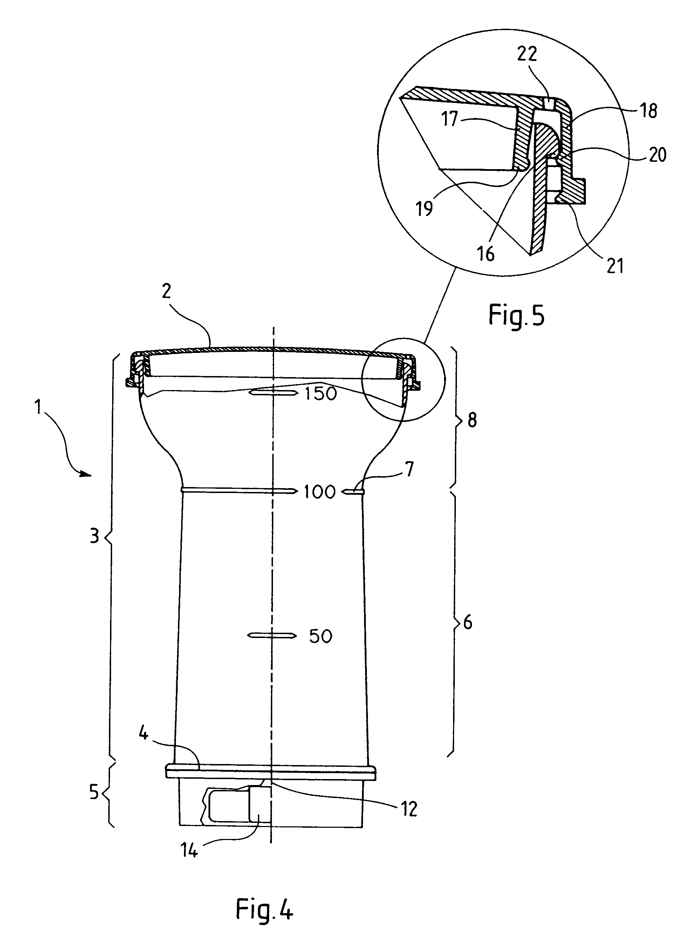

Referring to figures 1 and 4, the frustoconical portion (6) of the upper part of the container is extended by a funnel-shaped section (8) widening from the smallest diameter (7) of the container towards the open upper end (9) whose diameter is greater than the smallest diameter (7) and which comprises an outer circular ring (16) whose function will be explained later.

The volume of the frustoconical section (6) is equal to the culture medium volume recommended by the various pharmacopeia for incubating a culture medium, the surface of the liquid culture medium introduced into the container in accordance with the invention being located at the level of the smallest diameter of the container.

The funnel shape of the section (8) of the container facilitates manual introduction of the sample to be filtered and rinsing because it is flared outwardly to a diameter which is preferably 1.5 times that of the smallest inside diameter; this shape of the section (8) also minimises the surface area of contact with the culture medium, in particular that required to detect anaerobic micro-organisms.

The lower part (5) of the container is fastened to a drainage support (10) comprising an outer circular ring (11) and a series of concentric annular channels and radiating radial channels discharging into a central drainage orifice (12) and delimited by a series of ribs which support the microporous filter membrane (4); the central drainage orifice (12) may be sealed using a small plug 14 with gripping lugs.

Before it is welded, the hydrophilic microporous filter membrane (4) may be in the form of a continuous strip or a precut disk. Whichever form is used, said filter membrane is preferably heat welded at its periphery to the circular ring 13 of the frustoconical section (6) of the container.

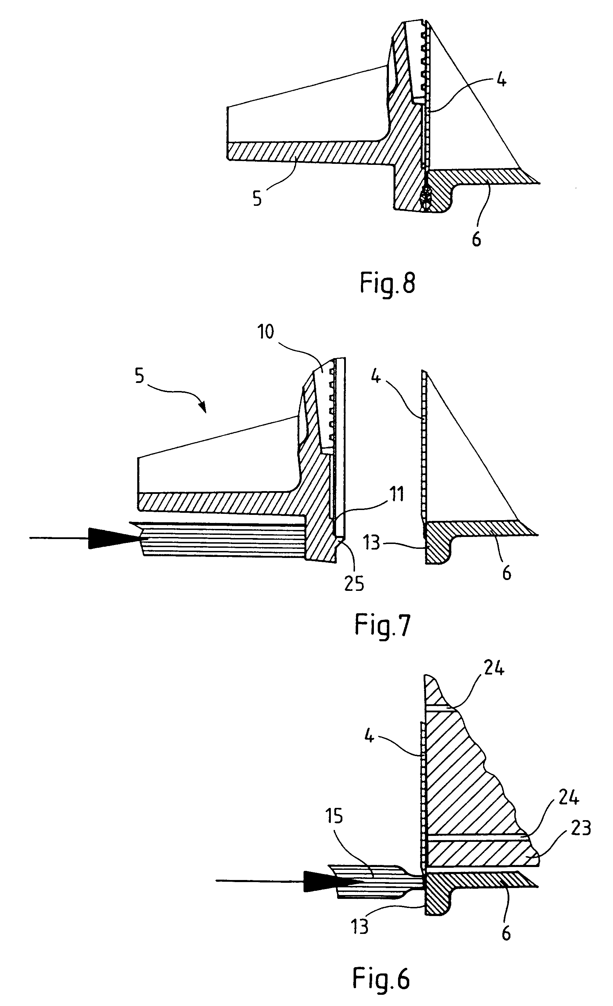

In the embodiment shown in figure 6 the filter membrane (4) is in the form of a precut disk. The disk is held in place on a perforated support (23) inserted into the upper part (3) of the container by suction generated by creating a vacuum in the orifices 24 of the support 23.

The disk-shaped filter membrane can then be welded to the lower edge of the upper part of the container using a welding tool (15). After welding it is flat and taut.

It should be noted that the use of precut disk filter membranes (4) is not possible in the methods described in the prior art French patent 2 540 992 since in the latter the containers are entirely enclosed.

The lower part (5) of the container fastened to the drainage support (10) has a cuneiform energy director circular bead (25) on its outer ring (11). The diameter of this bead is greater than the diameter of the filter membrane (4).

The ring (13) of the upper part (3) of the container to which the filter membrane (4) has been welded is brought into contact with the ring (11) of the lower part (5) of the container and the energy director bead (25) is heated by any appropriate means (in particular by ultrasonic means) to melt the plastics material of the container and to weld the two parts of the container together on a diameter which is greater than that of the filter membrane (4).

Because of this double weld, antibiotics contained in the sample placed in the upper part of the container during filtering of the sample through the filter membrane (4) cannot infiltrate the weld between the two parts of the container because the filter membrane is fastened to the upper part (3) and its pores have been blocked at this location during the heat welding of the membrane to the frustoconical section (6) of this part.

This avoids all risk of growth of the micro-organisms to be detected being inhibited by antibiotic residues at the periphery of the membrane which would otherwise falsify the results of the sample sterility check.

The microporous filter membrane is a polymer material preferably selected from vinylidene polyfluoride and polycarbonate and has a mean pore diameter of 0.45 µm to retain all micro-organisms likely to be contained in the sample to be analysed and which can be detected by incubation in an appropriate culture medium using the techniques specified in national pharmacopeia.

The plastic material from which the upper and lower parts of the container are made is a rigid transparent plastic material and may be selected from polystyrene or styrene and acrylonitrile resins.

The container (1) just described may be closed by a lid (2) which is made from a flexible plastic material such as polyethylene or an elastomer.

The lid (2) comprises a system for attaching it to the open upper end (9) of the container (1) in two different positions respectively shown in figures 3 and 5.

The generally circular lid (2) comprises at its periphery two concentric axial circular skirts (17 and 18) extending downwards. They have different axial lengths, the outer skirt (18) being longer than the inner skirt (17).

The inner skirt (17) has an outside sealing lip (19) at its end and the outer skirt (18) has two inside fastening lips, one lip (20) facing the sealing lip (19) of the skirt (17) and the other lip (21) at the end of the skirt (18).

The lid (2) comprises two vent holes (22) at opposite ends of a common diameter of the lid and lying between the two concentric skirts (17 and 18) in the annular ring defined by the two skirts.

During filtering of the fluid sample whose sterility is to be checked, the lid (2) is in the position shown in figure 3 with the open upper end (9) of the container engaged between the two skirts (17 and 18) of the lid. The outside circular ring (16) of the container is held between the two fastening lips (20 and 21) of the skirt (18).

During vacuum filtering of the sample the necessary air can enter the container through the two vents (22) to maintain gaseous equilibrium between the interior of the container and the sterile ambient air.

Following filtering of the sample and rinsing, the central drainage orifice (12) is sealed with the small plug (14) with gripping lugs, the culture medium is poured into the top part of the container and the lid (2) is pressed fully down onto the outwardly flared upper part (8) of the container.

Incubation of the culture medium takes place in this position, shown in figure 5. The outside circular ring (16) of the container lies between the skirts (17 and 18) having passed successively over the first fastening lip (21) and then the second fastening lip (20) of the outside skirt (18). The sealing lip (19) of the inner skirt (17) is in contact with the inside of the container.

As incubation proceeds the air inside the container is expanded because of the temperature increase to the incubation temperature. This deforms the lid (2) which lifts the sealing lip (19) away from the container to release the excess pressure which can escape through the vent holes (22) without allowing air to enter from the exterior, the second fastening lip (20) holding the lid (2) in place.

This system of fitting the lid to the container avoids the need for a filter membrane at the vent holes (22) and regulates the expulsion of excess pressure during incubation under aseptic conditions.

## Claims

1. Device for checking the sterility of a fluid sample in particular of an antibiotic, comprising a transparent rigid plastics material container (1) including an upper part (3), a disk-shape hydrophilic microporous filter membrane (4) and a lower part (5) fastened to a drainage support, the two parts of the container and the microporous filter membrane disposed between the lower edge of the upper part and the upper edge of the lower part of the container being welded together in a fluid-tight manner at their peripheries, characterised in that the upper part (3) of the container has a frustoconical section (6) whose base delimits the filter surface area of the membrane (4) and whose other end has a smaller diameter (7), and a funnel-shape part (8) widening from the smaller diameter end (7) of the frustoconical part to an open upper end (9) whose diameter is greater than said smaller diameter, and in that said open upper end (9) of the upper part of the container is adapted to be closed by a flexible plastics material lid (2) adapted to be deformed by the pressure inside the container during incubation and provided with at least one vent hole (22) to vent excess pressure without contamination from the outside.

2. Device according to claim 1 characterised in that in use the surface of a liquid culture medium to be incubated inside the container is at the level of the smaller inside diameter end (7) of the frustoconical section (6).

3. Device according to claim 1 or claim 2 characterised in that the microporous filter membrane (4) is heat welded to a ring (13) at the lower edge of the upper part (3) of the container (1) and the lower part (5) of the container fastened to the drainage support (10) is welded to the same ring on a diameter greater than that of the filter membrane (4).

4. Device according to claim 3 characterised in that the lower part (5) is welded to the upper part (6) of the container by means of an energy director bead (25) fastened to the lower part (5) and whose diameter is greater than that of the filter membrane (4).

5. Device according to any one of the preceding claims characterised in that the microporous filter membrane (4) is made from a plastics material selected from vinylidene polyfluoride or polycarbonate.

6. Device according to any one of the preceding claims characterised in that the open upper end (9) of the container has an exterior circular ring (16) adapted to cooperate with at least one circular interior fastening lip (20 or 21) provided on a peripheral skirt (18) of the lid (2).

7. Device according to claim 6 characterised in that the lid (2) comprises two concentric axial circular skirts at its periphery, the inner skirt (17) being provided at its end with an exterior sealing lip (19) adapted to cooperate with the inside wall of the open upper end of the container and the outer skirt (18) being provided at its end with an interior circular fastening lip (21).

8. Device according to claim 7 characterised in that the outer circular skirt (18) at the periphery of the lid (2) comprises two circular interior fastening lips (20 and 21) adapted to cooperate with the outside circular ring (16) at the upper end (9) of the container (1).

9. Device according to claim 7 or claim 8 characterised in that at least one vent hole (22) is provided in the circular ring of the lid (2) defined by the two concentric axial skirts (17 and 18) of the latter.

## Patentansprüche

1. Einrichtung zum Überprüfen der Sterilität einer flüssigen Probe, insbesondere eines Antibiotikums, mit einem durchsichtigen, festen Kunststoffbehälter (1), der einen oberen Teil (3), eine scheibenförmige, hydrophile, mikroporöse Filtermembran (4) und einen unteren Teil (5), der an einem Drainage-Halter befestigt ist, aufweist, wobei die zwei Teile des Behälters und die zwischen der unteren Kante des oberen Teils und der oberen Kante des unteren Teils des Behälters angeordnete mikroporöse Filtermembran an ihrem Umfang flüssigkeitsdicht zusammengeschweißt sind, **dadurch gekennzeichnet**, daß der obere Teil (3) des Behälters einen kegelstumpfförmigen Abschnitt (6), dessen Boden den Filterflächenbereich der Membran (4) begrenzt und dessen anderes Ende (7) einen kleineren Durchmesser hat, und ein trichterförmiges Teil (8) aufweist, das sich von dem Ende (7) mit kleinerem Durchmesser des kegelstumpfförmigen Teils zu einem oberen offenen Ende (9) hin, dessen Durchmesser größer als dieser kleinere Durchmesser ist, aufweitet und daß dieses obere offene Ende (9) des oberen Teils des Behälters durch einen biegsamen Kunststoffdeckel (2) verschließbar ist, der durch den Innendruck des Behälters während einer Inkubation verformbar ist und mit mindestens einem Entlüftungsloch (22) zum Abführen von Überdruck ohne Verunreinigung von außen her ausgestattet ist.

2. Einrichtung nach Anspruch 1, dadurch gekennzeichnet, daß sich bei Gebrauch die Oberfläche eines flüssigen Kultur-Mediums, das im Behälter inkubiert werden soll, auf der Höhe des Endes (7) mit kleinerem Durchmesser des kegelstumpfförmigen Abschnitts (6) befindet.

3. Einrichtung nach Anspruch 1 oder Anspruch 2, dadurch gekennzeichnet, daß die mikroporöse Filtermembran (4) an einen an der unteren Kante des oberen Teils (3) des Behälters (1) liegenden Ring (13) warm angeschweißt und der untere, am Drainage-Halter (10) befestigte Teil (5) des Behälters an demselben Ring angeschweißt ist, und zwar auf einem Durchmesser, der größer als der der Filtermembran (4) ist.

4. Einrichtung nach Anspruch 3, dadurch gekennzeichnet, daß der untere Teil (5) mittels einer Energieleitwulst (25), die am unteren Teil (5) befestigt und deren Durchmesser größer als der der Filtermembran (4) ist, an den oberen Teil (6) des Behälters angeschweißt ist.

5. Einrichtung nach einem der vorhergehenden Ansprüche, dadurch gekennzeichnet, daß die mikroporöse Filtermembran (4) aus einem Kunststoff hergestellt ist, der aus Vinyliden-Polyfluorid oder -Polycarbonat ausgewählt ist.

6. Einrichtung nach einem der vorhergehenden Ansprüche, dadurch gekennzeichnet, daß das offene obere Ende (9) des Behälters einen äußeren Kreisring (16) zum Zusammenwirken mit mindestens einer kreisförmigen inneren Befestigungs-Lippe (20 oder 21), die auf einem Umfangsrand (18) des Deckels (2) vorgesehen ist, aufweist.

7. Einrichtung nach Anspruch 6, dadurch gekennzeichnet, daß der Deckel (2) an seinem Umfang zwei konzentrische, axiale, kreisförmige Ränder aufweist, wobei der innere Rand (17) an seinem Ende mit einer äußeren Dichtlippe (19) zum Zusammenwirken mit der Innenwand des offenen oberen Endes des Behälters und der äußere Rand (18) an seinem Ende mit einer inneren kreisförmigen Befestigungs-Lippe (21) versehen ist.

8. Einrichtung nach Anspruch 7, dadurch gekennzeichnet, daß der äußere kreisförmige Rand (18) am Umfang des Deckels (2) zwei kreisförmige, innere Befestigungslippen (20 und 21) zum Zusammenwirken mit dem außen liegenden Kreisring (16) am oberen Ende (9) des Behälters (1) aufweist.

9. Einrichtung nach Anspruch 7 oder Anspruch 8, dadurch gekennzeichnet, daß mindestens ein Entlüftungsloch (22) in dem Kreisring des Deckels (2) vorgesehen ist, der durch die zwei konzentrischen axialen Ränder (17 und 18) des letzteren festgelegt wird.

## Revendications

1. Dispositif de vérification de la stérilité d'un échantillon de fluide, en particulier un antibiotique, comportant un récipient (1) en matière plastique rigide transparente comprenant une partie supérieure (3), une membrane filtrante microporeuse hydrophile (4) en forme de disque et une partie inférieure (5) fixée à un support d'écoulement, les deux parties du récipient et la membrane filtrante microporeuse disposée entre le bord inférieur de la partie supérieure et le bord supérieur de la partie inférieure du récipient étant soudées entre elles d'une manière étanche au fluide à leurs périphéries, caractérisé en ce que la partie supérieure (3) du récipient comporte une section tronconique (6) dont la base délimite l'aire de la surface filtrante de la membrane (4) et dont l'autre extrémité présente un diamètre inférieur (7), et une partie (8) en forme d'entonnoir s'élargissant depuis l'extrémité (7) de diamètre inférieur de la partie tronconique jusqu'à une extrémité supérieure ouverte (9) dont le diamètre est supérieur audit diamètre inférieur, et en ce que ladite extrémité supérieure ouverte (9) de la partie supérieure du récipient est conçue pour être fermée par un couvercle (2) en matière plastique flexible conçu pour être déformé par la pression à l'intérieur du récipient pendant une incubation et pourvu d'au moins un trou d'évent (22) pour évacuer un excédent de pression sans contamination depuis l'extérieur.

2. Dispositif selon la revendication 1, caractérisé en ce que, lors de l'utilisation, la surface du milieu liquide de culture devant être incubé à l'intérieur du récipient est au niveau de l'extrémité (7) de diamètre intérieur plus petit de la section tronconique (6).

3. Dispositif selon la revendication 1 ou la revendication 2, caractérisé en ce que la membrane filtrante microporeuse (4) est thermosoudée à une bague (13) située à l'extrémité inférieure de la partie supérieure (3) du récipient (1) et la partie inférieure (5) du récipient fixée au support (10) d'écoulement est soudée à la même bague sur un diamètre supérieur à celui de la membrane filtrante (4).

4. Dispositif selon la revendication 3, caractérisé en ce que la partie inférieure (5) est soudée à la partie supérieure (6) du récipient au moyen d'une arête (25) d'orientation d'énergie fixée à la partie inférieure (5) et dont le diamètre est plus grand que celui de la membrane filtrante (4).

5. Dispositif selon l'une quelconque des revendications précédentes, caractérisé en ce que la membrane filtrante microporeuse (4) est formée d'une matière plastique choisie entre du polyfluorure de vinylidène et un polycarbonate.

6. Dispositif selon l'une quelconque des revendications précédentes, caractérisé en ce que l'extrémité supérieure ouverte (9) du récipient comporte une bague circulaire extérieure (16) conçue pour coopérer avec au moins une lèvre intérieure circulaire (20 ou 21) de fixation prévue sur une jupe périphérique (18) du couvercle (2).

7. Dispositif selon la revendication 6, caractérisé en ce que le couvercle (2) comporte deux jupes circulaires axiales concentriques à sa périphérie intérieure, la jupe intérieure (17) étant pourvue, à son extrémité, d'une lèvre extérieure (19) d'étanchéité conçue pour coopérer avec la paroi intérieure de l'extrémité supérieure ouverte du récipient et la jupe extérieure (18) étant pourvue à son extrémité d'une lèvre circulaire intérieure (21) de fixation.

8. Dispositif selon la revendication 7, caractérisé en ce que la jupe circulaire extérieure (18) à la périphérie du couvercle (2) comporte deux lèvres intérieures circulaires (20 et 21) de fixation conçues pour coopérer avec la bague circulaire extérieure (16) à l'extrémité supérieure (9) du récipient (1).

9. Dispositif selon la revendication 7 ou la revendication 8, caractérisé en ce qu'au moins un trou d'évent (22) est prévu dans l'anneau circulaire du couvercle (2) défini par les deux jupes axiales concentriques (17 et 18) de ce dernier.
